# EUROPEAN PATENT APPLICATION

(11) **EP 3 406 407 A1**
(43) Date of publication of application: **28.11.2018**
(21) Application number: 17173044.3
(22) Date of filing: 26.05.2017
(51) Int. Cl.: B25J 9/16, G05B 19/408

(54) **ROBOT SYSTEM WITH ADAPTIVE MOTION PERFORMANCE**

(71) Applicant: Syddansk Universitet, 5230 Odense M (DK); Georg-August-Universität, 37073 Göttingen (DE)
(72) Inventor: Nielsen, Jacob, 5230 Odense M (DK); Christensen, Thomas Søndergaard, 5210 Odense NV (DK); Kulvicius, Tomas, 37073 Göttingen (DE)
(74) Representative: Plougmann Vingtoft a/s

(57) **Abstract**

The invention relates to a robot system and a control system for controlling motion of the robot. The control system is capable of controlling motion of the robot, i.e. an end-effector of the robot, according to a predefined or determined motion path. The control system is further capable of adjusting the motion path into an adjusted motion path based on measured or otherwise determined interaction parameters which describes the relationship of the end-effector with the surrounding environment. The motion path may be described by a set of motion primitives. A specific use of the robot system, according to an aspect of the invention, is a robotic training system for assisting patients with performing training exercises.

## Description

### FIELD OF THE INVENTION

The invention relates to control of robot systems, particularly to control of the motion of the end-effector. The invention also relates to robot systems for training of patients with robot aided motion support.

### BACKGROUND OF THE INVENTION

Stroke victims may suffer from long-term disability and reduced mobility. Rehabilitation after a stroke is a demanding task for the patient and a costly challenge for both society and healthcare systems. The stroke patient's mobility and control of muscles may be improved by repetition of training exercise which causes the brain to re-learn control of the paralysed muscle groups. The repetition of training exercises requires several hours of assistance from a therapist.

Due to the nature of the training exercise with several repetitions of same exercises, e.g. repeated motions of the patients arm, the rehabilitation costs are high.

There have been made attempts to use robotic systems for assisting in the patient in performing the training exercises.

EP3085351 A1 discloses exercise robot suitable for rehabilitation. The disclosed invention concerns in particular a method in which the exercise robot learns an exercise movement on the basis of movements conducted by the aid of a human assistant holding the leg of a patient and moving said leg with muscular form to conduct an exercise movement. The rehabilitation robot actively accompanies the exercise movement in an active compliance mode and records the movement so as to determine an exercise movement stored in the control unit of the device. The rehabilitation robot can then produce the determined exercise in an exercise mode.

Robots or other machines suitable for rehabilitation needs to have certain properties so that the patient perceives the robot as an attractive alternative to human therapists. Accordingly, the robot needs to be in possession of an ability to adapt to the patient's physical performance and the variance of the exercises performed by different patients and by a given patient over time.

Accordingly, there is a need for improving mechanical machines such as robots so that they can be used successfully for assisting patients in performing training exercises, or for rehabilitation in general. Thus, it is a problem that existing industrial robots does not have capabilities to be used as training robots.

The inventors have realised that there is also a need for improving industrial robots for industrial use with respect to capabilities to perform motions of the end-effector which can be adapted gradually, e.g. dependent on surroundings where the end-effector moves.

### SUMMARY OF THE INVENTION

It is an object of the invention to address one or more of the above-mentioned problems with existing training machines in order to use mechanical machines or robots for assisting patients in performing training exercise. Besides the possibility of improving existing solutions, it is also an object of the of the invention to address other problems with use of mechanical devices for patient training as a supplement to the traditional use of therapists. It is also an object of the invention to improve industrial robots capabilities to perform motions and adapt motions dependent on surroundings.

In a first aspect of the invention, there is provided robot system with adaptive motion control, where the robot system comprises:
- a motorized multi-degree of freedom motion system which is connectable with an end-effector,
- a detector arranged to measure a least one interaction parameter indicative of an interaction between the end-effector and an object,
- a mathematical description of a motion path for the end-effector as a function of the at least one interaction parameter,
- a control system arranged to control the end-effector to move along the motion path, and arranged to
- to determine an adjusted motion path based on the at least one interaction parameter and by use of the mathematical description so that the motion of the end-effector can be controlled to change from the motion path to the adjusted motion path.

The mathematical description enables determination of an adjustment of a motion path currently used by the control system into an adjusted motion path dependent on the interaction parameter, e.g. dependent of contact, force, orientation or other relationships between end-effector and the object. As an example, the mathematical description may be based on dynamic motion primitives or other mathematical models which provides a functionality to modify a mathematically defined n-dimensional motion path into an adjusted mathematically defined m-dimensional motion path, where m and n may be equal or different. The mathematical description may be based on motion or shape primitives from which the motion path and adjusted motion path can be mathematically described.

Accordingly, the mathematical description may define a relationship between the motion path for the end-effector, e.g. the actual motion path, and an adjusted motion path obtained as an adjustment of the motion path, where the relationship is a function of the at least one interaction parameter. In other words, the mathematical description can be seen as a mathematical description of adjustable motion paths which are adjustable as a function of the at least one interaction parameter.

The object may refer to any object such as surroundings e.g. an interior of a tunnel in which the end-effector moves. For example, the robot may be a surgical robot where the motion of end-effector needs to adapt to tissue of a patient as the end-effector, e.g. a cutting device, moves through the tissue, e.g. vessels. The object could also refer to moveable arbitrarily placed and oriented objects which the robot should detect and move. Accordingly, the robot may need to move along an adjusted motion path in order to enable correct picking of the object.

To control the end-effector to move along the motion path may be achieved by determination of control input for the motorized motion system where the control input can be processed by the motorized motion system to move according to the motion path. Accordingly, the control system may be arranged to determine the control input. The control input for the motorized motion system can have various formats dependent on what control input is required by the motorized multi-degree of freedom motion system for controlling actuators thereof. The control input is determined as a function of the motion path or any adjustment thereof, e.g. by use of a mathematical relationship between the motion path and the control input. This mathematical relationship may be comprised by the mathematical description.

Advantageously, due to the capability of adjusting the motion path based on the at least one interaction parameter and by use of the mathematically defined motion path, the motion of the end-effector can be adapted to unforeseen changes in the surroundings where the end-effector moves. Accordingly, the adjustment may be performed during motion of the end-effector.

A further aspect of the invention relates to a control system arranged to control motion of the end-effector of the motorized multi-degree of freedom motion system and to determine the adjusted motion path of a robot system based on the at least one interaction parameter in accordance with the first aspect.

According to an embodiment, the at least one interaction parameter comprises one or more of a force or torque acting on the end-effector due to an interaction with the object, a distance between the end-effector and the object, a relative velocity or an acceleration between the end-effector and the object and an orientation of the object relative to the end-effector.

According to an embodiment the motion path is described by one or more motion primitives of the mathematical description. Advantageously, by describing the motion path using motion primitives, the motion path can be described by a combination of motion primitives which are relevant for the actual motions to be performed by the end-effector. For example, the adaption may involve inclusion of a motion primitive to enable a specific motion required according to the interaction parameters. Specific interaction parameters may be associated beforehand to specific motion primitives so that adaption of the motion path is simplified.

According to an embodiment, the mathematical description enables determination of the adjusted motion path by adjustment of the one or more motion primitives of the motion path. For example, the adjustment may involve adjustment of the amplitude or curvature of a curved motion primitive, angular range of a rotation, a slope of a linear motion and any combination thereof.

According to an embodiment, the mathematical description enables determination of the adjusted motion path so that the adjusted motion path comprises one or more motion primitives not comprised by the motion path. In this way, the degrees of freedom in terms of available motion primitives can be adjusted as needed by adding, removing or replacing motion primitives from the motion path.

According to an embodiment, the control system is arranged to control the motion of the end-effector by successive adjustments of the motion path based on successive determinations of the interaction parameter to enable the motion of the end-effector to adapt to the object as the end-effector approaches the object. Accordingly, the motion path can be adjusted gradually during motion of the end-effector, e.g. due to changes of the interaction parameter.

According to an embodiment, the robot system is arranged as a robotic training system for training a limb of patient. Advantageously, due to the capabilities of the robot to adapt the motion path dependent on the interaction parameter, the motion path of a training exercise can be adapted dependent on the performance of the patient.

The robotic training system may be utilized for training of different body parts such as arms and legs and for different reasons such as post-stroke rehabilitation.

According to an embodiment, the robot system is configured as a robotic training system so that
- the motorized motion system is arranged to provide motorized support of a motion performed by the patient via a patient interface arranged as the end-effector,
- the detector is arranged to measure a least one training parameter indicative of load acting on the patient interface and/or a velocity or acceleration of the patient interface, and
- the control system is arranged to control the patient interface to support the motion performed by the patient along the motion path or the adjusted motion path.

Advantageously, since the motion path can be adapted gradually to the needs of the patient, the initial motion path used when the patient starts an exercise can be a standard motion path retrieved e.g. from a database. Accordingly, recording of a motion path for the patient is not required. Therefore, the patient can start the exercise with only little preparation and without need for a therapist to setup an initial motion path.

Furthermore, the capability of adjusting the motion path implies that differences between patients such as different arm lengths, different heights and different patient-positions relative to the patient interface can be compensated due to the adaptive capabilities.

When the desired motion path is described by a combination of motion primitives, the control of the motion of the patient interface or the end-effector, can be achieved by determining the resulting motion path from the combination of motion primitives or the by controlling the motorized motion system to perform the each of the motion primitives simultaneously, i.e. by processing the motion primitives as references for the motion in parallel.

According to an embodiment the control system is arranged to the determine the adjusted motion path based on the at least one training parameter. Accordingly, based on one of the training parameters or a combination thereof, the motion path can be adjusted. In this way, if the patient has difficulties with performing a given motion path the force provided by the patient on the patient interface may be very low. This could indicated that the motion path is too demanding and, therefore, the motion path could be modified into a less demanding path, e.g. a path with decreased travel length or reduced complexity.

According to an embodiment, the control system is arranged to adjust the motion path based on a progression parameter determined based on a history of the training parameters. The adjustment of the motion path may be performed at a tempo which is adapted to the performance of a patient. Accordingly, if the training parameter shows a faster improvement of e.g. the force delivered by the patient than an average expectation, the progression parameter may be changed so that the adjustment of the motion path is performed at a larger tempo than average.

According to an embodiment, the robot system has a recording mode for determining the motion path as an initial motion path by recording the motion of the end-effector. Advantageously, the use of motion primitives enables a simple determination of an initial motion path by fitting the motion primitives to the recorded motion of the end-effector. The position and motion data of the recorded motion may be obtained from position sensors of the motorized motion system.

According to an embodiment, the control system is arranged to control the patient interface to support the motion performed by the patient by providing an assisting or opposing force along the motion path or the adjusted motion path.

According to an embodiment, the control system is arranged to enable setting of different assisting or opposing forces for different motion primitives. According to this embodiment, an assisting force or low opposing force for can be set for a motional demanding motion primitive simultaneously with a normal opposing force for a less demanding motion primitive.

According to an embodiment, the control system is arranged to adjust the assisting or opposing force for at least one motion primitive dependent on the at least one training parameter. Accordingly, in the same way as a therapist would adapt the guidance or motion-resistance to the patient, the robotic system can adapt forces dependent on the measured performance of the patient.

According to an embodiment, the control system is arranged to adjust the assisting or opposing force dependent on a location or orientation of the patient interface relative to the motion path or adjusted motion path. In this way, similar to how a therapist could adapt the guidance or motion-resistance to the patient, the robotic system can adapt forces dependent on how demanding the motion at different locations is. The dependency of the forces on the location or orientation may be set manually, be predefined according to the motion primitives or the dependency can be obtained automatically dependent on the measured training parameters or interaction parameters.

In summary the invention relates to a robot system and a control system for controlling motion of the robot. The control system is capable of controlling motion of the robot, i.e. an end-effector of the robot, according to a predefined or determined motion path. The control system is further capable of adjusting the motion path into an adjusted motion path based on measured or otherwise determined interaction parameters which describes the relationship of the end-effector with the surrounding environment. The motion path may be described by a set of motion primitives. A specific use of the robot system according to an aspect of the invention is a robotic training system for assisting patients with performing training exercises.

In general, the various aspects and embodiments of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
Fig. 1 shows a robot system with a motorized multi-degree of freedom motion system and an end-effector,
Fig. 2A shows a principal illustration of a set of motion primitives and a motion path composed by one or more motion primitives,
Fig. 2B shows a motion path with attractor points and alternative paths of the preferred motion path,
Fig. 3 shows an example of a robotic training system which is based on the robot system of Fig. 1,
Fig. 4A shows an example of an adjusted motion path, and
Fig. 4B shows an example of a deviating path from the intended motion path.

### DETAILED DESCRIPTION

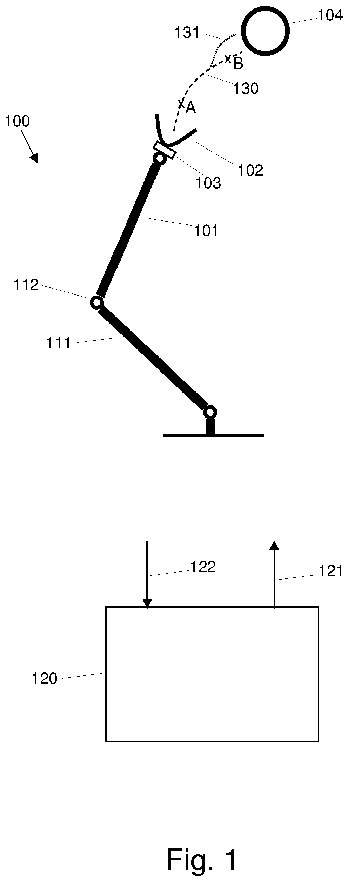

Fig. 1 shows robot system 100 which comprises a motorized multi-degree of freedom motion system 101 and an end-effector 102.

The motorized multi-degree of freedom motion system 101 may comprise a linkage system provided electric motors for rotating elements of the linkage system relative to each other. Accordingly, the multi-degree of freedom motion system 101 may be an industrial robot.

The linkage system may as illustrated comprise rigid elements 111 and motion actuators 112 capable of rotating and/or displacing the rigid elements 111 or other elements such as the end-effector 102. The rotation provided by one actuator 112 may be about any one or more axes such as an axis perpendicular or parallel to the longitudinal extension of a rigid element 111.

The multi-degree of freedom motion system 101 would have at least two degrees of freedom, preferably at least three degrees of freedom.

The end-effector 102 may be a gripper, a robotic hand, a tool such as a cutting or welding tool. The end-effector 102 could also be a patient interface such as a handle allowing a patient to use the robot system 100 as a training or exercise machine. The end-effector could also be an interface such as a bracket to which tools or other devices can be attached.

The robot system 100 further comprises a detector 103 arranged to measure a least one interaction parameter 122 indicative of an interaction between the end-effector and an object 104.

As an example, the detector 103 may be a sensor capable of measuring forces and/or torques acting on the end-effector 102. Accordingly, the detector may be a force-torque sensor located between the end-effector and a rigid element 111 or a motion actuator 112.

Other possible detectors include a distance detector capable of determining a distance, relative velocity or acceleration between the end-effector 102 and the object 104a. Other examples include vision sensors such as image sensors or laser scanners cable of determining orientation, dimension or other characteristics of the object relative to the end-effector. Accordingly, the interaction parameter 122 may include one or more of these data or characteristics of the object 104 or between the end-effector 102 and the object 104.

The robot system 100 further comprises a control system 120 arranged to control the end-effector to move along a motion path 130 by determination of control input 121 for the motorized motion system 101, i.e. control output from the control system 120.

The control input 121 may be in the form of control parameters for each of the actuators 112, e.g. control parameters which is used as a positional reference for controlling motion of the actuators 112. The control input 121 could have other formats, e.g. as a reference motion of the end-effector 102 which is received by a controller of the motorized multi-degree of motion system 101 and used by this controller for controlling the actuator 112. Thus, the motorized motion system 101 may be controlled by forward or inverse kinematics. Accordingly, the control input 121 may describe the motion path 130 by specific control parameters for the actuators 112 or by a desired motion path of the end-effector 102. In the current setup, the control input is in the form of the motion path 130.

The motion path 130 describes the desired motion of the end-effector 102, e.g. from point A to point B. The motion path 130 may not only describe the motion in 2D or 3D space of the end-effector 102, but may also include rotations of the end-effector which does not affect the position and velocities and accelerations of the end-effector 102.

The control system 120 is further arranged to determine an adjusted motion path 131 based on the at least one interaction parameter 122 and by use of a mathematical description so that the motion of the end-effector can be controlled to change from the first motion path 130 to the adjusted motion path 131.

The mathematical description is arranged to encode and/or modify the motion path 130, i.e. to determine an adjusted motion path 131, based on the at least one interaction parameter 122. In addition, the mathematical description may include a relationship between the motion path or the modified motion path and control input for the motorized motion system 101. Thus, the mathematical description may contain a first part arranged to determine the motion path or the adjusted motion path 130,131 and a second part arranged to determine the control input 121 based on the determined motion path 130, 131. Accordingly, the mathematical description is a function of the at least one interaction parameter 122. As previously noted, the control input may be equivalent or identical to the determined motion path or adapted motion path in which case the second part of the mathematical description may be omitted or reduced to a simple conversion of the motion path 130 to the control input 121.

The control system 120, other computer processors or a combination thereof, may be arranged to process the mathematical description for determining the motion path 130, the adjusted motion path 131 and the control input 121. Accordingly, the control system 120 may receive the at least one interaction parameter 122 as an input.

The determination of the adjusted motion path 131 may be performed during movement of the end-effector, i.e. in real time. Alternatively, the adjusted motion path 131 may be determined as an adjustment at a given point in time, but which is used at a later point in time - e.g. for the purpose of modifying or replacing a motion path 130 which is performed or supported repeatedly by the end-effector at any suitable subsequent time.

For example, the object 104 may be an apple on a tree and the end-effector may be a gripper arranged to pick apples. In order to pick the apple, the angle of attack of the end-effector 102 relative to the apple needs to certify certain requirements. The end-effector 102 is controlled initially along predetermined motion path 130, i.e. an initial motion path 130. As the gripper comes closer to the apple, the interaction parameter 122 obtained by the detector 103, e.g. an interaction parameter 122 in the form of an image or an orientation of the apple, may indicate a need for a change of the angle of attack. Since the mathematical description includes a specification of the optimal angle of attack, the motion path 130 is modified in response to the determined interaction parameter into an adjusted motion path 131 which has an improved or optimal angle of attack.

The adjustment of the motion path 130 may be performed at predetermined times or on-line in response to changes in the interaction parameter, and/or dependent on other conditions such as the distance between the end-effector 102 and the object 104.

According to an embodiment of the present invention the motion path 130 and consequently also the adapted motion path 131 is described by one or more motion primitives.

Any one of motion primitives defines a specific shape for the end-effector 102 or joint 112, e.g. a linear motion, curved motion, circular or elliptic motion and rotations of the end-effector about an axis of the end-effector. The motion primitives may be described in 2D or 3D coordinates including polar rotation coordinates. Motion primitives may have associated velocities or accelerations for the defined motion path and adjustments of the motion path.

The motion primitives may be described by low-level primitives which are associated to each of the degrees of freedom of the motorized motion system 101. In general there is at least one low-level motion primitive for each degree of freedom of the motorized motion system 101. Thus, a 6-DOF system 101 has at least six low-level primitives. Accordingly, a curved 3D motion path 130 will be described by at least one of low-level primitive for each DOF of the system. An adjustment of a motion primitive is therefore described as change of one or more low-level primitives.

Accordingly, the motion path 130 and the adjusted motion path 131 can be described as a combination of one or more of the motion primitives from the set of basic motion primitives. Advantageously, the mathematical description constitutes an adaptive or dynamic control algorithm capable of changing the motion path by use of motion primitives in response to effects from the surroundings, i.e. the interaction parameters, where the change of the motion path is formulated by changes in the motion primitives or low-level primitives.

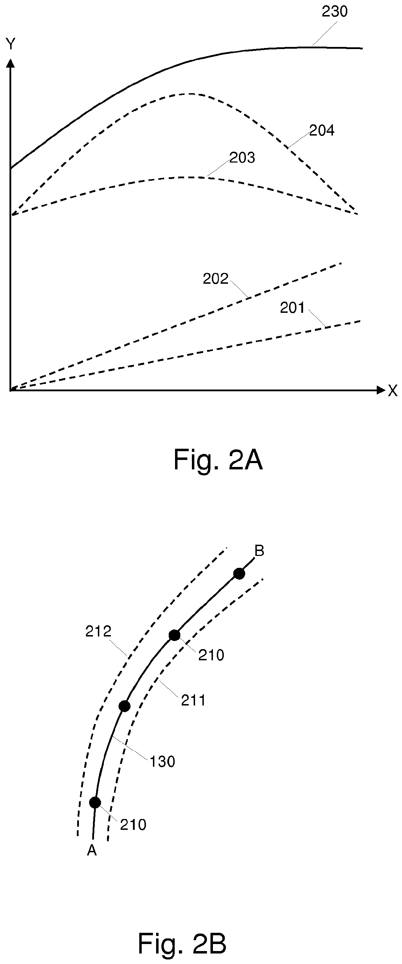

Fig. 2A shows an illustration of a set of motion primitives 201-204 in spatial coordinates x and y. The linear motions 201 and 202 are generated from the same basic (linear) motion primitive, but with different end-points (and/or different start points in another example). Alternatively, one of the linear motions (e.g. 201) could be a linear motion primitive and the other (e.g. 202) could be a linear motion determined from the linear motion primitive. Similarly, the curved motions 203 and 204 are generated from the same basic (curved) motion primitive, but with different scaling factors. Alternatively, one of the curved motions (e.g. 203) could be a curved motion primitive and the other (e.g. 204) could be a curved motion determined from the curved motion primitive. Thus, the set of motion primitives need only one of each basis motion primitives which can be used to generate different motion of similar shape by scaling and/or changing start- and/or end-points.

Fig. 2A also shows motion path 230 obtained as a linear combination of the linear motion primitives 201 and the curved motion primitive 203 with appropriate scaling factors and new start- and end-points.

The scaling factors and adjustments of these can be determined by use of the mathematical description analogue to the determination of motion paths 130, 131. Accordingly, an adapted motion path 131 may be obtained by adaption of scaling factors of the motion primitives 201-204 of the motion path 130 and/or start- and/or end-points. Thus, in general the adjusted motion path 131 may be obtained by adjustment of the one or more motion primitives 201-204 of the motion path, e.g. by adjustment of scaling factors, start- and end-points, or other shape describing parameters, such as eccentricity of elliptic shapes.

Furthermore, the adjustment of the motion path 130 may include adjustment of the a velocity or speed and/or an acceleration of the end-effector 102 along the motion path. Accordingly, an adjustment of the motion path 130 could imply only and adjustment of speed without changes of shape or start/end-point.

In addition to or alternative to adjusting the motion path 130 using scaling factor or other shape describing parameters, the motion path 130 may be adjusted by including new motion primitives 201-204 into the adjusted motion path 131 and/or by removing or replacing motion primitives 201-204 of the previous motion path 130. Accordingly, the motion path 130 and the adjusted motion path may include different and different numbers of motion primitives 201-204.

Accordingly, the mathematical description may be arranged to adjust the motion path 130 so that the adjusted motion path 131 may include one or more motion primitives not included by the motion path 130 or previously adjusted motion path 130.

The motion of the end-effector may be controlled in steps so that a new adjusted motion path 131 for a subsequent step is determined by an adjustment of the planned motion path at the next step based on the interaction parameter 122 at the current time step. Clearly, the motion path 130 for the next step will not be changed if the interaction parameter at the current step does not require a modification of the motion. In general, the motion of the end-effector may be controlled by successive adjustments of the motion path, if required, based on successive determinations of the interaction parameter 122. In this way, the motion of the end-effector can be adapted to the object 104 as the end-effector approaches the object. In other words, the motion can be adapted to the surroundings of the environment in which the end-effector operates.

In the context of successive adjustments of the motion path 130, it is clear that the motion path 130 may be a first motion path in a present step, and the adjusted motion path 131 may be a second, possibly adjusted, motion path in a subsequent step.

Fig. 2B shows a motion path 130 from location A to location B. The points 210 on the path denote attractor points located along the path. For each point on the motion path 130 there exist a number of alternative paths, e.g. 211, 212.

If the end-effector 102 for some reason has been displaced from the preferred motion path 130 to a point on one of the alternative paths 211, 212, the motion of the end-effector will be attracted by the attractor points so that motion along one of the alternative paths is gradually changed to motion along the preferred motion path 130.

For example, in the case where end-effector 102 is a gripper arranged to pick apples, the motorized multi-degree of freedom motion system 101 may be affected by wind so that the motion of the gripper is forced away from the desired motion path 130 to an alternative motion path 211. However, due to the attractor points 210, the motion of the gripper is changed back to the preferred motion path 130. During the motion along the alternative motion paths 211, 212 or the preferred motion path 130, the motion as described by the actual motion path is realised by determination of the control input 121.

According to an embodiment, the robot system 100 is configured as a robotic training system for rehabilitation of a patient, specifically for training a limb of a patient, e.g. an upper limb. For example, the robot system 100 may be used for post-stroke and nerve injury rehabilitation.

For the purpose of rehabilitation the motion path 130 can act as a basis for training exercise that can be modified into individualized and adaptive rehabilitation exercises that fit with the patient's physical properties and impairments. The exercises can be adapted to specific patients and furthermore to the progress of a patients physical capabilities.

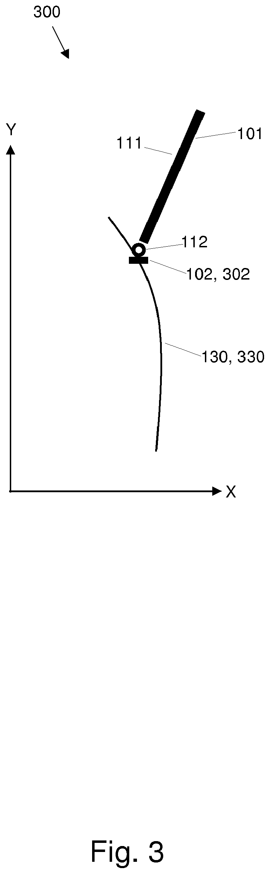

Fig. 3 illustrates an example of an embodiment where the robot system 100 is configured as a robotic training system 300. The motorized motion system 101 is arranged to provide motorized support of a motion path 330 performed by the patient via a patient interface 302.

Fig. 3 illustrates a part of the motorized multi-degree of freedom motion system 101, where the end-effector 102 is a patient interface 302 such as a handle. The motion path 330 corresponds in principles to the motion path 130 in Fig. 1.

For example, the patient may perform a simple back and forth motion of the underarm, i.e. where the patient moves the patient interface 302 back and forth in the x-y plane. The hand of the patient may be attached to the handle, e.g. using Velcro, in case the patient is not able to hold the handle. The back and forth motion could be completely linear or elliptical as shown.

The support of a motion path 330 provided by the motorized motion system 101 may be in the form of an assisting force which assists the muscle-force provided by patient or which guides the patient's hand. Alternatively, the support may be in the form of an opposing force which counteracts the force provided by the patient. Accordingly, the force provided by the patient interface 302 may be varied from a positive assisting force to a negative opposing force and could also be set to a substantial zero-force so that the patient neither experiences an assisting for nor an opposing force.

The control system 120 controls the patient interface to support the motion performed by the patient along the motion path 330 or an adjusted motion path. Accordingly, the control system 120 controls the force provided by the patient interface 302, e.g. as a predetermined force or an adaptive force which is adapted dependent on the performance of the patient. The force may be varied during the motion along the motion path 330, e.g. dependent on the position along the motion path 330. For example, the patient interface 302 provides an opposing force near the center of the back and forth motion and an assisting force near the end-points of the motion path 330 where the patient's muscle force is weaker.

Accordingly, the control system 120 may be arranged to adjust the assisting or opposing force dependent on a location or orientation of the patient interface relative to the motion path or adjusted motion path. In this way the supporting forces can be adapted to the capabilities of the patient, or to motivate for force the patient to perform specific motions. For example, the patient may be helped to move the patient interface 302 to the endpoints of the motion path, e.g. by applying an assisting force toward the end-point.

The detector 103 in the robotic training system is arranged to measure at least one training parameter 122 indicative of load acting on the patient interface and/or a velocity or acceleration of the patient interface. The training parameter is equivalent to the interaction parameter 122. Thus, the force detector provides information of the force generated through the interaction between the motorized motion system 101 and the patient. Accordingly, the detector may be a force-torque detector or a velocity/acceleration detector similarly to the examples for the general robot system 100. The detector is not illustrated in Fig. 3 for convenience. The force-torque detector or a velocity/acceleration detector does not need to be a specifically designed force-torque or velocity/acceleration detector. For example, the force and torques can be determined from currents in the electric motors used for rotating elements of the motorized motion system 101. Similarly, the velocity/acceleration can be determined from accelerations of the electric motors, e.g. from encoders. Accordingly, the detector 103 may be embodied by existing components of the motorized motion system 101. This applies both to the general robot system 100 and the robotic training system.

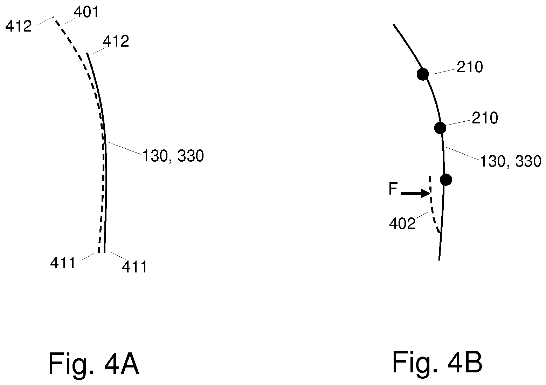

Fig. 4A illustrates an example where the motion path 330 has been adjusted into an adjusted motion path 401. The adjustment may involve modification of start and/or stop points 411, 412, the relative weights of e.g. a curved motion primitive and a linear motion primitive in a linear combination of these primitives or other modifications as described. The control system may be arranged to determine the adjusted motion path 401 based on the interaction parameter 122 (training parameter 122). For example, if the patient is able to deliver a push or pull force at the end-points of the motion path 330 which is sufficient high, e.g. above a force threshold, the length of the motion path may be adjusted as illustrated. The adjustment may be performed gradually during the motion or the adjustment may be performed after an evaluation of the performance of the patient.

For example, the adjustment of the motion path 330 may be based on a history of the at least one interaction parameter. For example, if the average of the determined force values near the end-points or if the average of velocities near the end-points or other locations is above a given threshold, the control system 220 automatically performs the adjustment or the control system 220 may provide a suggestion to the patient or a therapist to adjust the motion path.

In this way, the adjustment of the motion path 330 corresponds to an adjustment which could have been decided traditionally by a therapist based on observations of the patient's performance.

The motion path 330 is described by one or more motion primitives 201-204 as previously explained. For example, the motion path 330 could be described by one or more of a straight translation of the arm (linear motion), a rotation of the wrist (rotation about an axis of the patient interface 302) and curved motion of the hand.

As described above, the adjustment of the motion path 330 may involve adjustment of various shape parameters of the motion primitives 201-204 and addition, removal or replacement of motion primitives of the motion path 330. For example, the wrist rotation may be removed from the motion path 330 in case the training parameters show insufficient torque force for performing the wrist rotation.

As explained above, the supporting assisting or opposing force provided by the motorized motion system may be different for different locations along the motion path 330. Similarly, the supporting assisting or opposing force may be different for different motion primitives. The supporting force for individual motion primitives may set manually or automatically by the control system 120. Thus, the control system 120 may be arranged to enable setting of the assisting or opposing forces dependent on the motion primitives 201-204 comprised by the motion path 330.

For example, a motion path 330 may comprise a linear motion primitive and a wrist rotation motion primitive, where the control system 120 is controlled to generate an opposing force for the linear motion primitive and an assisting force for the wrist motion primitive. In this way, the patient can train muscles for the back and forth motion, whereas the wrist rotation is guided in order to promote control of the wrist rotation.

According to an embodiment, the control system is arranged to adjust the assisting or opposing force for at least one motion primitive 201-204 dependent on the at least one training parameter.

For example, the assisting or opposing force for one or more motion primitives could be adjusted dependent on the force provided by the patient for a different or the same motion primitive. For example, an opposing force for a given motion primitive could be reduced or changed to an assisting force, if the force or velocity provided by the patient is below a threshold or is decreasing. As another example, the opposing force for one motion primitive such as a linear motion primitive could be decreased if the patient provides a wrist rotation force. This type of control, would motivate the patient to train his wrist muscles.

The adjustment of the motion path 330 may be performed dependent on a progression parameter. The progression parameter may be set manually or determined as an adaptive progression parameter by the robot system 100 based on the training parameters 122. For example, the progression parameter may be determine as a function of the speed of the motion of the patient interface 302 so that e.g. the amplitude of the motion path 330 is increased more rapidly if the progression parameter is large due to a rapid speed progression of the patient.

According to an embodiment, the robot system has a recording mode for recording an initial motion path by recording the trajectory of the end-effector. The initial motion path can be used as motion path 330 for the patient. The recording may be performed by determining the position of the patient interface 302 during the movement of the patient interface 302 (e.g. by recording position or encoder data of the motorized motion system 101). The motion of the patient interface 302 may be performed by the patient, the therapist or other person. Based on the position data, the initial motion path can be determined by fitting the motion primitives 201-204 to match the position data.

Fig. 4B shows an example where the motion of the patient interface 302 due to a force provided by the patient deviates along path 402 from the desired motion path 330 (alternative motion paths 211, 212). In order to control the deviations of the motion performed by the patient, the control system 120 can be arranged to generate an opposing force F for deviations from the desired motion path. The opposing force F prevents too large deviations and guides the motion performed by the patient back to the desired motion path 330. The control of the opposing force F may be controlled by use of the attractor points 210 by adjusting the opposing force F dependent on deviation, i.e. distance, from the desired trajectory, or by use of a constant force F pointing towards the attractor points 210. Accordingly, similar to the previous example of apple pick by use of a robot gripper, due to the attractor points 210, the motion of patient interface 302 is changed back to the preferred motion path 330.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A robot system (100) with adaptive motion control, the robot system comprises:
- a motorized multi-degree of freedom motion system (101) which is connectable with an end-effector (102),
- a detector (103) arranged to measure a least one interaction parameter (122) indicative of an interaction between the end-effector and an object (104),
- a mathematical description of a motion path (130, 330) for the end-effector as a function of the at least one interaction parameter,
- a control system (120) arranged to control the end-effector to move along the motion path, and arranged to
- to determine an adjusted motion path (131, 401) based on the at least one interaction parameter and by use of the mathematical description so that the motion of the end-effector can be controlled to change from the motion path to the adjusted motion path.

2. A robot system according to claim 1, where the at least one interaction parameter comprises one or more of a force or torque acting on the end-effector due to an interaction with the object, a distance between the end-effector and the object, a relative velocity or an acceleration between the end-effector and the object and an orientation of the object relative to the end-effector.

3. A robot system according to any of the preceding claims, where the motion path is described by one or more motion primitives (201-204) of the mathematical description.

4. A robot system according to claim 3, where the mathematical description enables determination of the adjusted motion path by adjustment of the one or more motion primitives of the motion path.

5. A robot system according to any of claims 3-4, where the mathematical description enables determination of the adjusted motion path so that the adjusted motion path comprises one or more motion primitives not comprised by the motion path.

6. A robot system according to any of the preceding claims, where the control system is arranged to control the motion of the end-effector by successive adjustments of the motion path based on successive determinations of the interaction parameter to enable the motion of the end-effector to adapt to the object as the end-effector approaches the object.

7. A robot system according to any of the preceding claims, where the robot system is arranged as a robotic training system (300) for training a limb of a patient.

8. A robot system according to claim 7, where
- the motorized motion system is arranged to provide motorized support of a motion performed by the patient via a patient interface (302) arranged as the end-effector,
- the detector (103) is arranged to measure at least one training parameter (122) indicative of load acting on the patient interface and/or a velocity or acceleration of the patient interface, and
- the control system (120) is arranged to control the patient interface to support the motion performed by the patient along the motion path or the adjusted motion path.

9. A robot system according to any of claims 7-8, where the control system is arranged to the determine the adjusted motion path (401) based on the at least one training parameter.

10. A robot system according to claim 7-9, where the control system is arranged to adjust the motion path based on a progression parameter determined based on a history of the training parameters.

11. A robot system according to any of claims 7-10, where the robot system has a recording mode for determining the motion path (330) as an initial motion path by recording a trajectory of the patient interface (302).

12. A robot system according to any of claims 7-11, where the control system is arranged to control the patient interface to support the motion performed by the patient by providing an assisting or opposing force along the motion path or the adjusted motion path.

13. A robot system according to claim 12, where the control system is arranged to enable setting of different assisting or opposing forces for different motion primitives.

14. A robot system according to any of claims 12-13, where the control system is arranged to adjust the assisting or opposing force for at least one motion primitive dependent on the at least one training parameter.

15. A robot system according to any of claims 12-14, where the control system is arranged to adjust the assisting or opposing force dependent on a location or orientation of the patient interface relative to the motion path or adjusted motion path.
